# EUROPEAN PATENT SPECIFICATION

(11) **EP 2 086 578 B1**
(45) Date of publication and mention of the grant of the patent: **11.01.2012**
(21) Application number: 07822693.3
(22) Date of filing: 19.11.2007
(51) Int. Cl.: A61K 39/08

(54) **USE OF CLOSTRIDIUM PERFRINGENS TYPE C BACTERIUM FOR THE MANUFACTURE OF A VACCINE**
VERWENDUNG VON CLOSTRIDIUM PERFRINGENS-TYP C-BAKTERIUM ZUR HERSTELLUNG EINES IMPFSTOFFS
UTILISATION D'UNE BACTÉRIE DE CLOSTRIDIUM PERFRINGENS TYPE C POUR LA FABRICATION D'UN VACCIN

(30) Priority: 20.11.2006 EP 06124404; 20.11.2006 US 860447 P
(43) Date of publication of application: 12.08.2009
(73) Proprietor: Intervet International B.V., 5831 AN Boxmeer (NL)
(72) Inventor: WITVLIET, Maarten Hendrik, 5831 AN Boxmeer (NL); SMEETS, Jozef Franciscus Maria, 5831 AN Boxmeer (NL); REDHEAD, Keith, Milton, Keynes MK7 7AJ (GB)
(74) Representative: Janssen, Paulus J. P.
(86) International application number: PCT/EP2007/062484
(87) International publication number: WO 2008/061950

(56) References cited:
- SELBITZ H J ET AL: "Immunoprophylaxis of clostridioses of piglets" TIERÄRTZL PRAX, vol. 28, no. 6, November 2000 (2000-11), pages 337-340, XP008084545
- CHERFAOUI S ET AL: "[Production of a vaccine against enterotoxemia from Clostridium perfringens strains isolated in the field]" ARCHIVES DE L'INSTITUT PASTEUR D'ALGÉRIE INSTITUT PASTEUR D'ALGÉRIE 1992, vol. 58, 1992, pages 205-224, XP008084535 ISSN: 0020-2460
- GRECO GRAZIA ET AL: "Clostridium perfringens toxin-types in lambs and kids affected with gastroenteric pathologies in Italy" VETERINARY JOURNAL, vol. 170, no. 3, November 2005 (2005-11), pages 346-350, XP002456188 ISSN: 1090-0233
- LUGINBUEHL A: "The necrotizing enteritis by Clostridium perfringens type C in piglets: Practical experience management and epidemiology." SCHWEIZER ARCHIV FUER TIERHEILKUNDE, VERLAG HANS HUBER, BERN, CH, vol. 144, no. 6, June 2002 (2002-06), pages 263-273, XP008084524 ISSN: 0036-7281

## Description

The present invention relates to the use of *Clostridium perfringens* type C bacterium for the manufacture of a vaccine.

Clostridia are Gram-positive spore-forming anaerobic bacteria. The Clostridia are widely recognized as pathogens of both domestic and wild animals. An overview of the various Clostridia and the animal diseases they cause is given by Songer, J.G. in The Clostridia; Chapter 10: Clostridial Diseases of Animals, Molecular biology and Pathogenesis, ed. Rood, J.I. et al., Academic Press Ltd (1997) ISBN 0-12-595020-9.

Of the currently known Clostridial species, *Clostridium perfingens* is sometimes considered to be the most widely occurring bacterial pathogen, and of all Clostridial species it certainly is the most important cause of Clostridial enteric disease in domestic animals.

The species *Clostridium perfingens* is sub-divided in five types; type A-E, on the basis of the major toxins they make.

*Clostridium perfingens* types A and C, and *Clostridium difficile* are the principal enteric clostridial pathogens of swine.

Clostridial enteric infections in pigs have more specifically been described by Songer, J.G. and Uzal, F.A. (J. Vet. Diagn. Invest 17: 528-536 (2005)).

*Clostridium perfringens* type C infects pigs, cattle, chickens, humans, sheep, dogs and horses. Piglets are the most vulnerable to *Clostridium perfringens* type C infection. In piglets 1-2 days old, the infection causes morbidity rates of between 30%-50% and case fatality rates of 50%-100%. Older piglets (1-2 weeks old) may have a longer course of infection.

Sows are the common source of infection.

The common approach for the protection of piglets is vaccination of sows with a *Clostridium perfringens* type C vaccine, in order to induce antibodies that are transferred to their offspring via colostrum. This leads to a high level of protection in piglets at that moment in time at which they are the most vulnerable to Clostridial infection. Such vaccines are based upon toxoids. Usually, they are detoxified supernatant vaccines, in which no cells are present. Toxoid vaccines have been described i.a. by Springer, S. and Selbitz, H.-J. (FEMS Immun. and Medical Microbiol. 24: 333-336 (1999)).

*Clostridium perfringens* type A infects lambs, goats, calves, chickens, pigs horses, dogs and humans. In fully developed pigs, *Clostridium perfringens* type A is a member of the normal flora of the intestine. It is therefore currently assumed that fully developed pigs are not adversely affected by the presence of *Clostridium perfringens* type A in the intestine.

It can however cause porcine necrotic enterocolitis in neonatal and weaned pigs. Vaccines for the protection of pigs against *Clostridium perfringens* type A are rare. There is currently only one (conditionally licensed) vaccine commercially available.

One of the reasons for this may be the fact that, as said above, *Clostridium perfringens* type A is a member of the normal flora. Therefore, one would not expect it to trigger the immune system.

As a consequence, one would not expect antibody-production to be induced against *Clostridium perfringens* type A. If antibodies were induced, *Clostridium perfringens* type A would not survive in the gut, let alone be a member of the normal flora. As a further consequence, one would of course not expect sows to transmit any protection to their offspring through the colostrum. For whatever reason, it seems that *Clostridium perfringens* type A does not induce sufficient immune stimulation. Selbitz et al. (Tierärztl Prax. 28(G) :337-340; (2000)) describe the use of herd-specific vaccines against C. perfringens type A infection.

It has now been surprisingly found, that *Clostridium perfringens* type C vaccines comprising detoxified culture supernatant are very capable of inducing cross-protection against *Clostridium perfringens* type A infection.

More surprising, it was found that vaccination of sows with *Clostridium perfringens* type C culture supernatant leads to a significant protection of their piglets against *Clostridium perfringens* type A infection.

Even more surprising, it was found that even the sows perform better after this vaccination. This is surprising, because it was generally assumed, as mentioned above, that fully developed pigs, such as sows are not adversely affected by *Clostridium perfringens* type A infection. This finding for the first time proves the opposite to be the case.

Therefore, a first embodiment of the present invention relates to the use of *a Clostridium peefringens* type C bacterium for the manufacture of a vaccine for the protection of fully developed pigs against Clostridium perfringens type A infection.

A second embodiment of the present invention relates to the use of a *Clostridium perfringens* type C bacterium for the manufacture of a vaccine for the protection of sows against Clostridium perfringens type A infection.

A third embodiment of the present invention relates to the use of a *Clostridium perfringens* type C bacterium for the manufacture of a vaccine for sows, for the protection of piglets against Clostridium perfringens type A infection.

A fourth embodiment of the present invention relates to the use of a *Clostridium perfringens* type C bacterium for the manufacture of a vaccine for the protection of piglets against Clostridium perfringens type A infection.

As the skilled artisan will understand, in fact the use of a *Clostridium perfringens* type C bacterium for the manufacture of a vaccine is a two step process: the *Clostridium perfringens* type C bacterium is used for growth in a culture medium, in order to obtain the culture supernatant that is the basis for the manufacture of the vaccine according to the invention.

Therefore, another embodiment of the present invention relates to the use of a *Clostridium perfringens* type C culture supernatant for the manufacture of a vaccine for the protection of fully developed pigs against Clostridium perfringens type A infection.

Still another embodiment of the present invention relates to the use of a *Clostridium perfringens* type C culture supernatant for the manufacture of a vaccine for the protection of sows against Clostridium perfringens type A infection.

Again another embodiment of the present invention relates to the use of a *Clostridium perfringens* type C culture supernatant for the manufacture of a vaccine for sows, for the protection of piglets against Clostridium perfringens type A infection.

Also an embodiment of the present invention relates to the use of a *Clostridium perfringens* type C culture supernatant for the manufacture of a vaccine for the protection of piglets against Clostridium perfringens type A infection.

Within the *Clostridium perfringens* type C toxin type, a division can be made between those strains that produce beta-2 toxin and those strains that don't. About 40% of all *Clostridium perfringens* type C strains produce this toxin.

It was found that a *Clostridium perfringens* type C bacterium that produces beta-2 toxin is even more suitable than a non-beta-2 toxin producing *Clostridium perfringens* type C bacterium. Such strains and their identification are i.a. described by Fisher, D.J. et al. (Inf. & Immun. 74: 5200-5210 (2006)).

Thus, a preferred form of this embodiment relates to a use wherein a *Clostridium perfringens* type C bacterium is used that produces beta-2 toxin.

Vaccines manufactured as described above are by no means the only vaccines that are administered to pigs in the process of commercial pig rearing. Clostridial vaccines are frequently given around the same time as vaccines against other pig pathogenic organisms and viruses.

Thus, in another preferred embodiment, the vaccine manufactured as described in the present invention additionally comprises one or more antigens derived from pig pathogenic organisms or viruses, antibodies against those antigens or genetic information encoding such antigens.

Such organisms or viruses are preferably selected from the group of Pseudorabies virus, PRRS virus, Porcine influenza virus, Porcine parvo virus, Transmissible gastro-enteritis virus, Rotavirus, *Escherichia coli, Erysipelothrix rhusiopathiae, Bordetella bronchiseptica, Salmonella typhimurium, Salmonella choleraesuis, Haemophilus parasuis, Pasteurella multocida, Streptococcus suis, Mycoplasma hyopneumoniae, Brachyspira hyodysenteriae* and *Actinobacillus pleuropneumoniae.*

Therefore, a more preferred form of this embodiment relates to a vaccine manufactured as described in the present invention that additionally comprises one or more antigens derived from pig pathogenic organisms or viruses, wherein these organisms or viruses are selected from the group of Pseudorabies virus, PRRS virus, Porcine influenza virus, Porcine parvo virus, Transmissible gastro-enteritis virus, Rotavirus, *Escherichia coli, Erysipelothrix rhusiopathiae. Bordetella bronchiseptica. Salmonella typhimurium, Salmonella choleraesuis, Haemophilus parasuis, Pasteurella multocida, Streptococcus suis, Mycoplasma hyopneumoniae, Brachyspira hyodysenteriae* and *Actinobacillus pleuropneumoniae.*

Next to *Clostridium perfringens* infection, piglets frequently suffer from *Escherichia coli* infection. *Escherichia coli* is the cause of another severe enteric disease in piglets. Therefore, piglets or pregnant sows are frequently vaccinated with *Escherichia coli* vaccines. E. coli vaccines are commonly used in the field and they are commercially available.

A combination vaccine comprising both *Clostridium perfringens* type C culture supernatant and *Escherichia coli* vaccine components therefore is highly desirable, because it protects against *Clostridium perfringens* type C, *Clostridium perfringens* type A and *Escherichia coli* infection.

Therefore, a most preferred form of this embodiment relates to a vaccine manufactured as described in the present invention that additionally comprises one or more antigens derived from the pig pathogenic organism *Escherichia coli.*

The invention is exemplified in the following examples.

### Examples.

### Production of Clostridium perfringens type C antigen

A standard *Clostridium perfringens* type C strain, strain 587 (also named CWC 1/S, from the Weybridge U.K. Collection) was cultured in a fermentor until the beginning of the stationary growth phase. The culture was centrifuged and the pellet containing the bacterial cells was discarded. Formaldehyde was added to the supernatant to a final concentration of 0.5% v/v to inactivate toxins, followed by concentration by means of ultra filtration and 0.2 µm filtration.

### Vaccine preparation

A vaccine was formulated by mixing the *Clostridium perfringens* type C antigen and a tocopherol-based adjuvant. Each dose of vaccine (2 ml) comprised 20µl of a 17-fold concentrated *Clostridium perfringens* type C antigen toxoid culture supernatant concentrate.

The vaccine complied with the European Pharmacopoeia monograph 0363 (*Clostridium perfringens* vaccine for veterinary use) regarding safety and potency of type C vaccines.

Additionally, in this experiment purified *E. coli* antigens (F4ab, F4ac, F5 and F6 fimbriae and heat-labile toxin) were added to suppress possible *E*. *coli* infection. These components are known in the art, and the skilled artisan would know how to make a vaccine on the basis of these components.

Alternatively, however the skilled artisan could use a commercially available and ready-to-use *E*. *coli* vaccine, such as Porcilis Porcoli DF (obtainable through Intervet Int. B.V., Wim de Korverstraat 35, Boxmeer, The Netherlands).

The control vaccine comprises the same components except for the *Clostridium perfringens* type C antigen, that was not present in the control group.

### Protection of sows.

A Dutch pig production farm on which there was a clinical *Clostridium perfringens* type A outbreak was selected for a randomized double blinded field efficacy study. The *Clostridium perfringens* type A isolate from the farm was found to produce both α-toxin and β2-toxin.

Sows were vaccinated IM during pregnancy at approximately 6 and 2 weeks before farrowing with either a vaccine containing E. coli antigens only (control group) or with a vaccine containing E. coli antigens and *Clostridium perfringens* type C antigen (test group). The vaccines were color-coded (2x test vaccine, I x control vaccine) to assure blinding, and distribution of the sows over the groups was approximately 2:1 (Table 1).

**Table 1. Group sizes**

| Group | Number of sows | Number of live born piglets per sow | Total number of piglets |
|---|---|---|---|
| Control | 41 | 11.6 | 453 |
| Test | 77 | 12.1 | 904 |

From the day of first vaccination until the time of weaning of the piglets (3-4 weeks after farrowing), the sows were observed every 3 days for general health, feed intake and diarrhea. To facilitate statistical analysis, a clinical scoring system was used (Table 2). Piglets born to the vaccinated sows were observed daily during the first week of life, once every three days from one week until weaning and weekly thereafter up to the end of the nursery period. General health, feed intake, diarrhea and mortality were recorded (Table 2).

**Table 2 Scoring system used for sows and piglets:**

| Clinical Score | Demeanour | Appetite | Faecal Consistency | Faecal Composition |
|---|---|---|---|---|
| 0 | Normal | Normal | Normal | Normal |
| 1 | Depressed | Slight inappetance | Soft | Mucus present |
| 2 | Lethargic | Marked inappetance | Liquid | Mucus with blood present |
| 3 | Moribund | Not suckling | Watery | Blood present with intestinal casts |

### Results

**Table 3. Clinical observations of sows**

| Score (N) | | 0 | 1 | 2 | 3 | Mean |
|---|---|---|---|---|---|---|
| General | Control | 687 | *99* | *51* | *20* | *0.30* |
| Demeanour | Test | 1623 | 33 | 0 | 0 | 0.02 |
| Appetite | Control | *597* | *139* | *71* | *49* | *0.50* |
| | Test | 1598 | 47 | 9 | 0 | 0.04 |
| Faecal | Control | *385* | *250* | *153* | *68* | *0.89* |
| Consistency | Test | 1564 | 67 | 17 | 7 | 0.07 |
| Faecal | Control | *780* | *71* | *4* | *0* | *0. 09* |
| Composition | Test | 1647 | 8 | 0 | 0 | 0.01 |

Table 3 shows the effect of vaccination with a vaccine comprising the *Clostridium perfringens* type C antigens compared to vaccination with a vaccine without *Clostridium perfringens* type C antigens.

As follows unambiguously from Table 3, vaccination with the vaccine comprising the *Clostridium perfringens* type C antigens resulted in a very significant improvement of the health status of the sows.

### Protection of the offspring of vaccinated sows.

Also, the mortality rate in the liters of the sows that had been vaccinated with the vaccine comprising the *Clostridium perfringens* type C antigens was compared to that in the liters of sows that had been vaccinated with a vaccine without *Clostridium perfringens* type C antigens.

As follows unambiguously from Table 4, the mortality rate in the liters of the sows that had been vaccinated with the vaccine comprising the *Clostridium perfringens* type C antigens was significantly lower than in the control group.

In addition, Table 5 shows that piglets from sows that had been vaccinated with the vaccine comprising the *Clostridium perfringens* type C antigens had significantly lower clinical scores than the control group.

**Table 4. Weekly mortality in control and test litters**

| Age (days) | Control | Test |
|---|---|---|
| 0-6 | 29 | 22 |
| 7-13 | 3 | 3 |
| 14-21 | 7 | 2 |
| 21-27 | 6 | 1 |
| 28-34 | 2 | 1 |
| 35-41 | 0 | 0 |
| 42-48 | 1 | 1 |
| 49-55 | 1 | 1 |
| 56-62 | 1 | 0 |
| 63-70 | 0 | 0 |
| Total (%) | 50 (11.04) | 31 (3.43) |

**Table 5. Clinical observations of piglets**

| *Score* (N) | | 0 | 1 | 2 | 3 | Mean |
|---|---|---|---|---|---|---|
| General | Control | *336* | *232* | *137* | *73* | *0.93* |
| Demeanour | Test | 1159 | 271 | 55 | 11 | 0.28 |
| Appetite | Control | *426* | *166* | *128* | *58* | *0.77* |
| | Test | 1336 | 125 | 31 | 4 | 0.13 |
| Faecal | Control | *544* | *118* | *71* | *40* | *0.49* |
| Consistency | Test | 1419 | 48 | 17 | 12 | 0.08 |
| Faecal | Control | *636* | *104* | *31* | *6* | *0.24* |
| Composition | Test | 1414 | 70 | 10 | 1 | 0.06 |

Conclusion: the data show that vaccination of sows with a vaccine containing *Clostridium perfringens* type C antigens results in a significant improvement of the health status of both vaccinated sows and their offspring in face of a *Clostridium perfringens* type A outbreak.

## Claims

1. Use of a *Clostridium perfringens* type C culture supernatant for the manufacture of a vaccine for the protection of fully developed pigs against Clostridium perfringens type A infection.

2. Use of a *Clostridium perfringens* type C culture supernatant for the manufacture of a vaccine for the protection of sows against Clostridium perfringens type A infection.

3. Use of a *Clostridium perfringens* type C culture supernatant for the manufacture of a vaccine for sows for the protection of piglets against Clostridium perfringens type A infection.

4. Use of a *Clostridium perfringens* type C culture supernatant for the manufacture of a vaccine for the protection of piglets against Clostridium perfringens type A infection.

5. Use according to claim 1-4, **characterised in that** a *Clostridium perfringens* type C bacterium is used that produces beta-2 toxin.

6. Use according to claims 1-5, **characterised in that** for the manufacture of said vaccine additionally one or more antigens derived from pig pathogenic organisms or viruses, antibodies against those antigens or genetic information encoding such antigens are used.

7. Use according to claim 6, **characterised in that** said pathogenic organism or virus is selected from the group of Pseudorabies virus, PRRS virus, Porcine influenza virus, Porcine parvo virus, Transmissible gastro-enteritis virus, Rotavirus, *Escherichia coli, Erysipelothrix rhusiopathiae, Bordetella bronchiseptica, Salmonella choleraesuis, Salmonella typhimurium, Haemophilus parasuis, Pasteurella multocida, Streptococcus suis, Mycoplasma hyopneumoniae, Brachyspira hyodysenteriae* and *Actinobacillus pleuropneumoniae.*

8. Use according to claim 7, **characterised in that** said pig pathogenic organism is *Escherichia coli.*

## Patentansprüche

1. Verwendung eines Kulturüberstands von Clostridium perfringens Typ C zur Herstellung eines Impfstoffs zum Schutz ausgewachsener Schweine gegen eine Infektion mit Clostridium perfringens Typ A.

2. Verwendung eines Kulturüberstands von Clostridium perfringens Typ C zur Herstellung eines Impfstoffs zum Schutz von Säuen gegen eine Infektion mit Clostridium perfringens Typ A.

3. Verwendung eines Kulturüberstands von Clostridium perfringens Typ C zur Herstellung eines Impfstoffs für Säue zum Schutz von Ferkeln gegen eine Infektion mit Clostridium perfringens Typ A.

4. Verwendung eines Kulturüberstands von Clostridium perfringens Typ C zur Herstellung eines Impfstoffs zum Schutz von Ferkeln gegen eine Infektion mit Clostridium perfringens Typ A.

5. Verwendung nach Anspruch 1-4, **dadurch gekennzeichnet, dass** ein Beta-2-Toxin produzierendes Clostridium perfringens-Typ-C-Bakterium verwendet wird.

6. Verwendung nach Anspruch 1-5, **dadurch gekennzeichnet, dass** zur Herstellung des Impfstoffs zusätzlich ein oder mehrere aus für Schweine krankheitserregenden Organismen oder Viren stammende Antigene, Antikörper gegen diese Antigene oder solche Antigene codierende genetische Information verwendet wird bzw. werden.

7. Verwendung nach Anspruch 6, **dadurch gekennzeichnet, dass** der krankheitserregende Organismus bzw. das krankheitserregende Virus ausgewählt ist aus der Gruppe Pseudorabiesvirus, PRRS-Virus, Schweinegrippevirus, Schweineparvovirus, TGEV (Transmissible Gastro-enteritis Virus), Rotavirus, Escherichia coli, Erysipelothrix rhusiopathiae, Bordetella bronchiseptica, Salmonella choleraesuis, Salmonella typhimurium, Haemophilus parasuis, Pasteurella multocida, Streptococcus suis, Mycoplasma hyopneumoniae, Brachyspira hyodysenteriae und Actinobacillus pleuropneumoniae.

8. Verwendung nach Anspruch 7, **dadurch gekennzeichnet, dass** es sich bei dem für Schweine krankheitserregenden Organismus um Escherichia coli handelt.

## Revendications

1. Utilisation d'un surnageant de culture de *Clostridium perfringens* type C pour la fabrication d'un vaccin pour la protection de porcs totalement développés contre une infection à *Clostridium perfringens* type A.

2. Utilisation d'un surnageant de culture de *Clostridium perfringens* type C pour la fabrication d'un vaccin pour la protection de truies contre une infection à *Clostridium perfringens* type A.

3. Utilisation d'un surnageant de culture de *Clostridium perfringens* type C pour la fabrication d'un vaccin pour des truies pour la protection de porcelets contre une infection à *Clostridium perfringens* type A.

4. Utilisation d'un surnageant de culture de *Clostridium perfringens* type C pour la fabrication d'un vaccin pour la protection de porcelets contre une infection à *Clostridium perfringens* type A.

5. Utilisation selon les revendications 1 à 4, **caractérisée en ce qu'**une bactérie *Clostridium perfringens* type C est utilisée, qui produit la toxine bêta-2.

6. Utilisation selon les revendications 1 à 5, **caractérisée en ce que**, pour la fabrication dudit vaccin, un ou plusieurs antigènes dérivés d'organismes ou virus pathogènes chez le porc, des anticorps contre ces antigènes ou des informations génétiques codant pour de tels antigènes sont en outre utilisés.

7. Utilisation selon la revendication 6, **caractérisée en ce que** ledit organisme ou virus pathogène est choisi dans le groupe des virus de pseudo-rage, virus PRRS, virus de la grippe porcine, parvovirus porcin, virus de la gastro-entérite transmissible, rotavirus, *Escherichia coli, Erysipelothrix rhusiopathiae, Bordetella bronchiseptica, Salmonella choleraesuis, Salmonella typhimurium, Haemophilus parasuis, Pasteurella multocida, Streptococcus suis, Mycoplasma hyopneumoniae, Brachyspira hyodysenteriae* et *Actinobacillus pleuropneumoniae.*

8. Utilisation selon la revendication 7, **caractérisée en ce que** ledit organisme pathogène chez le porc est Escherichia coli.
